# EUROPEAN PATENT APPLICATION

(11) **EP 3 818 977 A1**
(43) Date of publication of application: **12.05.2021**
(21) Application number: 19207529.9
(22) Date of filing: 06.11.2019
(51) Int. Cl.: A61K 31/166, A61K 31/5415, A61P 35/02

(54) **CD93 INHIBITORS FOR USE IN THE TREATMENT OF CANCER**

(71) Applicant: Universität Bern, 3012 Bern (CH)
(72) Inventor: OCHSENBEIN, ADRIAN, 3032 Hinterkappelen (CH); RIETHER, CARSTEN, 3012 Bern (CH)
(74) Representative: Schulz Junghans Patentanwälte PartGmbB

(57) **Abstract**

The present invention relates to compounds of formula 1, Ar-L-N(R¹)(R²) (1), for use in the treatment of cancer. The compounds of formula 1 are characterized by a mono-, bi- or tricyclic aryl or heteroaryl (Ar) and a basic nitrogen atom with R¹ and R² being a linear alkyl or forming an aliphatic or heterocyclic ring. The aryl or heteroaryl and the basic nitrogen atom are connected via a linker L. The compounds of formula 1 are potent inhibitors of CD93, particularly of the CD93 intracellular domain.

## Description

### Field of the invention

The present invention relates to CD93 inhibitors such as metoclopramide and derivatives thereof for use in the treatment of cancer.

### Background of the invention

Haematological malignancies such as leukemias, lymphomas and myelomas account for 9.5% of new cancer diagnoses in the United States. Treatment ranges from "watchful waiting" and symptomatic treatment to chemotherapy, radiotherapy, immunotherapy and even bone marrow transplantation. As the five-year survival rate e.g. for leukemia is nevertheless below 60 % in the United States, there is still a need for novel drugs and treatment approaches.

The introduction of BCR/ABL-specific tyrosine kinase inhibitors (TKIs) more than two decades ago revolutionized chronic myelogenous leukemia (CML) therapy. The majority of CML patients treated with TKIs obtain durable cytogenetic and molecular responses. However, only a subgroup of these patients can successfully discontinue TKI therapy and maintain a treatment-free remission. TKI-resistant leukemia stem cells (LSCs) persist in the majority of patients at low levels over a prolonged period. These quiescent, self-renewing LSCs in the bone marrow (BM) are the major cause of relapse after drug discontinuation (Holyoake et al, 2017). The selective elimination of LSCs requires the definition of unique signaling pathways that promote self-renewal of LSCs but not of normal hematopoietic stem cells (HSCs). Based on the expression of CD93 on LSCs (Kinstrie et al, 2015), the present invention aims to provide inhibitors of the cell surface receptor CD93, which regulates the self-renewal of human and murine CML LSCs and contributes to disease development and progression.

Surprisingly, drugs that are commonly used to treat and prevent nausea and vomiting such as the dopamine-receptor antagonist *metoclopramide* or the antihistamine *promethazine* are potent CD93 inhibitors that are effective in reducing colony formation of murine LSCs.

Based on the above-mentioned state of the art, the objective of the present invention is to provide compounds for use in the treatment of cancer. This objective is attained by the subject-matter of the independent claims of the present specification.

### Description of the invention

### Terms and definitions

Unless defined otherwise, all technical and scientific terms used herein have the same meaning as commonly understood by one of ordinary skill in the art (e.g., in cell culture, molecular genetics, nucleic acid chemistry, biochemistry, pharmacy and medicine). Standard techniques are used for molecular, genetic and biochemical methods (see generally, Sambrook et al., Molecular Cloning: A Laboratory Manual, 2d ed. (1989) Cold Spring Harbor Laboratory Press, Cold Spring Harbor, N.Y. and Ausubel et al., Short Protocols in Molecular Biology (1999) 4th Ed, John Wiley & Sons, Inc.) and chemical methods.

The term gene refers to a polynucleotide containing at least one open reading frame (ORF) that is capable of encoding a particular polypeptide or protein after being transcribed and translated. A polynucleotide sequence can be used to identify larger fragments or full-length coding sequences of the gene with which they are associated. Methods of isolating larger fragment sequences are known to those of skill in the art.

The term *variant* refers to a polypeptide that differs from a reference polypeptide, but retains essential properties. A typical variant of a polypeptide differs in its primary amino acid sequence from another, reference polypeptide. Generally, differences are limited so that the sequences of the reference polypeptide and the variant are closely similar overall and, in many regions, identical. A variant and reference polypeptide may differ in amino acid sequence by one or more modifications (e.g., substitutions, additions, and/or deletions). A substituted or inserted amino acid residue may or may not be one encoded by the genetic code. A variant of a polypeptide may be naturally occurring such as an allelic variant, or it may be a variant that is not known to occur naturally.

Calculations of "homology" or "sequence identity" or "similarity" between two sequences (the terms are used interchangeably herein) are performed as follows. The sequences are aligned for optimal comparison purposes (e.g., gaps can be introduced in one or both of a first and a second amino acid or nucleic acid sequence for optimal alignment and non-homologous sequences can be disregarded for comparison purposes). In a particular embodiment, the length of a reference sequence aligned for comparison purposes is at least 30%, particularly at least 40%, more particularly at least 50%, even more particularly at least 60%, and even more particularly at least 70%, 80%, 90%, 100% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "homology" is equivalent to amino acid or nucleic acid "identity"). The percent identity between the two sequences is a function of the number of identical positions shared by the sequences, taking into account the number of gaps, and the length of each gap, which need to be introduced for optimal alignment of the two sequences. In the case of circularly related proteins, the sequence of one of the partners needs to be appropriately split and aligned in two sections to achieve optimal alignment of the functionally equivalent residues necessary to calculate the percent identity.

In the context of the present specification, the terms *sequence identity* and *percentage of sequence identity* refer to a single quantitative parameter representing the result of a sequence comparison determined by comparing two aligned sequences position by position. Methods for alignment of sequences for comparison are well-known in the art. Alignment of sequences for comparison may be conducted by the local homology algorithm of Smith and Waterman, Adv. Appl. Math. 2:482 (1981), by the global alignment algorithm of Needleman and Wunsch, J. Mol. Biol. 48:443 (1970), by the search for similarity method of Pearson and Lipman, Proc. Nat. Acad. Sci. 85:2444 (1988) or by computerized implementations of these algorithms, including, but not limited to: CLUSTAL, GAP, BESTFIT, BLAST, FASTA and TFASTA. Software for performing BLAST analyses is publicly available, e.g., through the National Center for Biotechnology-Information (http://blast.ncbi.nlm.nih.gov/).

One example for comparison of amino acid sequences is the BLASTP algorithm that uses the default settings: Expect threshold: 10; Word size: 3; Max matches in a query range: 0; Matrix: BLOSUM62; Gap Costs: Existence 11, Extension 1; Compositional adjustments: Conditional compositional score matrix adjustment. One such example for comparison of nucleic acid sequences is the BLASTN algorithm that uses the default settings: Expect threshold: 10; Word size: 28; Max matches in a query range: 0; Match/Mismatch Scores: 1.-2; Gap costs: Linear. Unless stated otherwise, sequence identity values provided herein refer to the value obtained using the BLAST suite of programs (Altschul et al., J. Mol. Biol. 215:403-410 (1990)) using the above identified default parameters for protein and nucleic acid comparison, respectively.

In the context of the present invention, the term "CD93" or "Cd93" relates to the receptor encoded by the murine gene ENSMUSG00000027435; *mCd93,* or the human CD93 gene (HGNC ID: 15855; Entrez Gene ID: 22918; Ensembl: ENSG00000125810; UniProtKB: Q9NPY3). Furthermore, the term "CD93" or "Cd93" includes homologs or variants thereof.

In the context of the present invention, the term "CD93 intracellular domain" or "Cd93^{intra}" lacks the sequence encoding for the extracellular domain of Cd93. The CD93 intracellular domain consists of the signal peptide (1-22aa), transmembrane (574-598aa) and cytoplasmic (599-644aa) domains of the Cd93 open reading frame (ORF) (total length of 282bp).

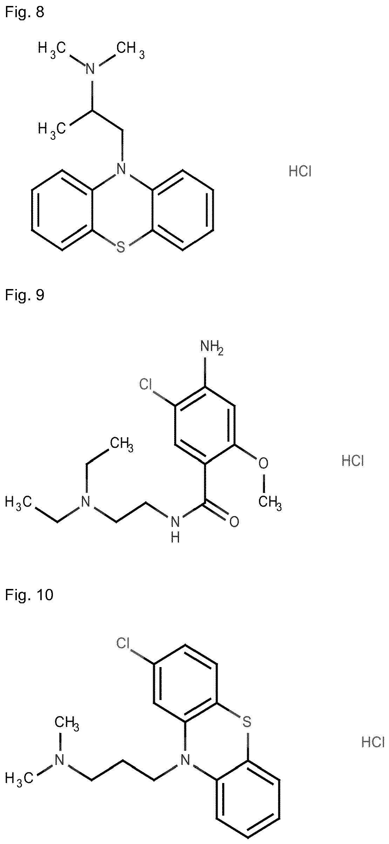

Promethazine (Cas No. 58-33-3) relates to the compound shown in Fig. 8.

Metoclopramide (Cas No. 7232-21-5) relates to the compound shown in Fig. 9.

Chlorpromazine (Cas No. 69-09-0) relates to the compound shown in Fig. 10.

A *C₁₋₆-alkyl* in the context of the present specification relates to a saturated linear or branched hydrocarbon having 1, 2, 3, 4, 5 or 6 carbon atoms. Non-limiting examples for a C₁-C₆ alkyl include methyl, ethyl, propyl, isopropyl, n-butyl, 2-methylpropyl, *tert*-butyl, n-pentyl, 2-methylbutyl, 3-methylbutyl, 1,1-dimethylpropyl, 1,2-dimethylpropyl, 3-methyl-2-pentyl, 4-methyl-2-pentyl and n-hexyl.

As used herein the term "alkenyl," refers to a straight or branched hydrocarbon chain moiety having at least one carbon-carbon double bond. Examples of alkenyl groups include, without limitation, ethenyl, propenyl, butenyl, 1-methyl-2-buten-1-yl, dienes such as 1,3-butadiene and the like.

As used herein the term "alkynyl," refers to a straight or branched hydrocarbon moiety having at least one carbon-carbon triple bond. Examples of alkynyl groups include, without limitation, ethynyl, 1-propynyl, 1-butynyl, and the like.

The term *fluoro substituted alkyl* or *alkyl substituted with -F* refers to an alkyl according to the above definition that is modified by one or several fluoride groups F. Non-limiting examples of fluoro-substituted alkyl include -CH₂F, -CHF₂, -CF₃, -(CH₂)₂F, -(CHF)₂H, -(CHF)₂F, -C₂F₅,-(CH₂)₃F, -(CHF)₃H, -(CHF)₃F, -C₃F₇, -(CH₂)₄F, -(CHF)₄H, -(CHF)₄F and -C₄F₉. Similarly, a fluoro substituted alkenyl or alkynyl refers to an alkenyl or alkynyl according to the above definition that is modified by one or several fluoride groups -F.

As used herein the term "aryl" refers to a hydrocarbon with alternating double and single bonds between the carbon atoms forming an aromatic ring structure, in particular a six (C₆) to 14 (C₁₄) membered ring or polyring structure. The term "heteroaryl" refers to compounds comprising a, in particular five to 14-membered, ring or polyring structure, comparable to aryl compounds, in which at least one member is an oxygen or a nitrogen or a sulphur atom. For example a 5-membered heteroaryl comprises a five membered ring structure with at least one carbon atom being replaced with an oxygen, a nitrogen or a sulphur atom. If the aryl or heteroaryl comprises more than one ring, the rings may be fused. Examples for a bicyclic aryl or heteroaryl comprising two fused rings are naphthaline or indol and derivatives thereof. Tricyclic aryls or heteroaryls having fused rings comprise for example anthracene or acridine.

### Description

The present invention relates to a compound of formula 1 for use in the treatment of cancer,

Ar-L-N(R¹)(R²) (1),

wherein
Ar is a mono-, bi or tricyclic aryl or heteroaryl, wherein Ar is unsubstituted or substituted with one or more substituents R³, wherein
   R³ is independently selected from
   -C₁₋₆-alkyl, -C₂₋₆-alkenyl, -C₂₋₆-alkynyl,
   -F, -Cl, -Br, -I, -CN,
   -NR^{a}₂, -C₁₋₆-alkyl-NR^{a}₂,
   -OR^{a}, -C₁₋₆-alkyl-OR^{a}
   -SR^{a}, -C₁₋₆-alkyl-SR^{a},
      with each R^{a} being selected independently from each other from H, a C₁₋₆ alkyl, a C₂₋₆ alkenyl, or a C₂₋₆ alkynyl,
   wherein the -C₁₋₆-alkyl, -C₂₋₆-alkenyl or -C₂₋₆-alkynyl is unsubstituted or halogen substituted, particularly unsubstituted or substituted with -F,
L is a linker,
N is a nitrogen atom,
R¹ and R² are independently selected from C₁₋₄-alkyl, or
R¹ and R² form a saturated aliphatic or heterocyclic ring.

Compounds of formula (1) are characterized by a mono-, bi or tricyclic aryl or heteroaryl forming an aromatic or heteroaromatic scaffold and a basic nitrogen atom that is connected to the scaffold via a linker.

In certain embodiments, R¹ and R² are independently selected from C₁₋₄-alkyl.

In certain embodiments, R¹ and R² are independently selected from methyl and ethyl.

In certain embodiments, the mono- bi- or tricyclic aryl or heteroaryl comprises one, two or three 5- or 6-membered rings.

In certain embodiments, the 5- or 6-membered rings of the bi- or tricyclic aryl or heteroaryl are fused. Examples for bicyclic aryls or heteroaryls comprising two fused rings are naphthaline or indol and derivatives thereof. Tricyclic aryls or heteroaryls having fused rings comprise for example anthracene or acridine.

In certain embodiments, Ar is a moiety of formula 2 or 3, 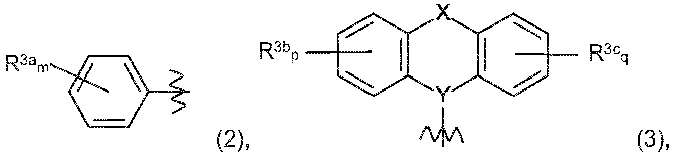 wherein
X is selected from -CH-, -NH-, -O- and -S-, particularly -O- and -S-, more particularly -S-,
Y is -N- or -CH-, particularly -N-,
R^{3a} R^{3b} and R^{3c} are independently selected as described above for R³,
m is 0, 1, 2, 3, 4 or 5, particularly 0, 1, 2 or 3, more particularly 1, 2 or 3,
p is 0, 1, 2, 3, 4, particularly 0, 1 or 2, more particularly 0 or 1,
q is 0, 1, 2, 3, 4, particularly 0, 1 or 2, more particularly 0 or 1.

In certain embodiments, the sum of p and q is ≤ 2.

In certain embodiments, the sum of p and q is ≤ 1.

In certain embodiments, R^{3a}, R^{3b} and R^{3c} are independently selected from
-C₁₋₄-alkyl, -C₂₋₄-alkenyl, -C₂₋₄-alkynyl,
-F, -Cl, -Br, -I, -CN,
-NR^{a}₂, -C₁₋₄-alkyl-NR^{a}₂,
-OR^{a}, -C₁₋₄-alkyl-OR^{a}
-SR^{a}, -C₁₋₄-alkyl-SR^{a},
   with each R^{a} being selected independently from each other from H, a C₁₋₄ alkyl, a C₂₋₄ alkenyl, or a C₂₋₄ alkynyl,
wherein the -C₁₋₄-alkyl, -C₂₋₄-alkenyl or -C₂₋₄-alkynyl is unsubstituted or substituted with -F.

In certain embodiments, R^{3a}, R^{3b} and R^{3c} are independently selected from
-C₁₋₄-alkyl, -C₂₋₄-alkenyl, -C₂₋₄-alkynyl,
-F, -Cl, -Br, -I,
-NR^{a}₂, -C₁₋₄-alkyl-NR^{a}₂,
-OR^{a}, -C₁₋₄-alkyl-OR^{a}
   with each R^{a} being selected independently from each other from H, a C₁₋₄ alkyl, a C₂₋₄ alkenyl, or a C₂₋₄ alkynyl,
wherein the -C₁₋₄-alkyl, -C₂₋₄-alkenyl or -C₂₋₄-alkynyl is unsubstituted or substituted with -F.

In certain embodiments, R^{3a}, R^{3b} and R^{3c} are independently selected from
-C₁₋₄-alkyl,
-F, -Cl, -Br, -I,
-NH₂, -C₁₋₄-alkyl-NH₂,
-OR^{a}, -C₁₋₄-alkyl-OR^{a}
   with each R^{a} being selected independently from each other from H, a C₁₋₄ alkyl,
wherein the -C₁₋₄-alkyl is unsubstituted or substituted with -F.

In certain embodiments, R^{3a}, R^{3b} and R^{3c} are independently selected from
-C₁₋₂-alkyl,
-F, -Cl, -Br, -I,
-NH₂, -C₁₋₂-alkyl-NH₂,
-OR^{a}, -C₁₋₂-alkyl-OR^{a}
   with each R^{a} being selected independently from each other from H, a C₁₋₂ alkyl,
wherein the -C₁₋₂alkyl is unsubstituted or substituted with -F.

In certain embodiments, R^{3a} is selected from -C₁₋₂-alkyl, -O-C₁₋₂-alkyl, -F, -Cl, -Br, -I, -NH₂, particularly -O-C₁₋₂-alkyl, -Cl, and -NH₂, and R^{3b} and R^{3c} are independently selected from -C₁₋₂-alkyl, -F, -Cl, -Br, -I, particularly methyl and -CI, more particularly -Cl.

In certain embodiments, R^{3a} is selected from -C₁₋₂-alkyl, -O-C₁₋₂-alkyl, -F, -Cl, -Br, -I, -NH₂, particularly -O-C₁₋₂-alkyl, -Cl, and -NH₂, or R^{3b} and R^{3c} are independently selected from -C₁₋₂-alkyl, -F, -Cl, -Br, -I, particularly methyl and -Cl, more particularly -Cl.

In certain embodiments, R^{3a} is selected from -C₁₋₂-alkyl, -O-C₁₋₂-alkyl, -F, -Cl, -Br, -I, -NH₂, particularly -O-C₁₋₂-alkyl, -Cl, and -NH₂.

In certain embodiments, R^{3b} and R^{3c} are independently selected from -C₁₋₂-alkyl, -F, -Cl, -Br,-I, particularly methyl and -Cl, more particularly -Cl.

In certain embodiments, the linker L has a length that corresponds to the length of a linear hydrocarbon chain comprising 12, particularly 8, more particularly 6 carbon atoms. Hereby, the linear hydrocarbon chain starts at the C atom that is bound to Ar and ends at the C atom that is bound to the basic nitrogen atom.

In certain embodiments, L is a linker comprising one or more moieties, particularly 1 to 4 moieties, independently selected from -C₁₋₆-alkyl, -O-, -NR⁴- and -C(=O)-, wherein R⁴ is H or-C₁₋₄-alkyl.

In certain embodiments, L is selected from -C₁₋₆-alkyl-, -NR⁴-C₁₋₆-alkyl-, -C(=O)-C₁₋₆-alkyl-,-C(=O)-NR⁴-C₁₋₆-alkyl-, -NR⁴-C(=O)-C₁₋₆-alkyl-, -[O-C₁₋₆-alkyl]ₙ-, -C₁₋₆-alkyl-[O-C₁₋₆-alkyl]ₙ-, wherein R⁴ is H or -C₁₋₄-alkyl, particularly H, methyl or ethyl, and n is 1, 2 or 3, particularly 1 or 2.

In certain embodiments, L is selected from -C₁₋₆-alkyl-, -NR⁴-C₁₋₆-alkyl-, -C(=O)-C₁₋₆-alkyl-,-C(=O)-NR⁴-C₁₋₆-alkyl-, -NR⁴-C(=O)-C₁₋₆-alkyl-, wherein R⁴ is H, methyl or ethyl, particularly H.

In certain embodiments, L is selected from -C₁₋₆-alkyl-, -C(=O)-NR⁴-C₁₋₆-alkyl-, -NR⁴-C(=O)-C₁₋₆-alkyl-, wherein R⁴ is H, methyl or ethyl, particularly H.

In certain embodiments, L is selected from -C₁₋₆-alkyl-, -C(=O)-NR⁴-C₁₋₆-alkyl-, wherein R⁴ is H, methyl or ethyl, particularly H.

In certain embodiments, L is selected from -C₁₋₄-alkyl-, -C(=O)-NR⁴-C₁₋₄-alkyl-, -NR⁴-C(=O)-C₁₋₄-alkyl-, wherein R⁴ is H, methyl or ethyl, particularly H.

In certain embodiments, L is selected from -C₁₋₄-alkyl-, -C(=O)-NR⁴-C₁₋₄-alkyl-, wherein R⁴ is H, methyl or ethyl, particularly H.

In certain embodiments, the compound of formula 1 is selected from promethazine, metoclopramide and chlorpromazine.

In certain embodiments, the compound of formula 1 is metoclopramide.

In certain embodiments, the compound of formula 1 is selected from promethazine and chlorpromazine.

In certain embodiments, the cancer is selected from leukemia, lymphoma and myeloma.

In certain embodiments, the cancer is selected from leukemia and myeloma.

In certain embodiments, the cancer is selected from acute lymphoblastic leukemia (ALL), acute myelogenous leukemia (AML), chronic lymphocytic leukemia (CLL), chronic myelogenous leukemia (CML), acute monocytic leukemia (AMoL), hairy cell leukemia, prolymphocytic leukemia (PLL), multiple myeloma (MM).

In certain embodiments, the cancer is selected from acute myelogenous leukemia (AML), chronic myelogenous leukemia (CML) and multiple myeloma (MM).

In certain embodiments, the cancer is CML.

Another aspect of the invention relates to a compound of formula (1) for use as a CD93 inhibitor, particularly as an inhibitor of the CD93 intracellular domain. Reference is made to the embodiments of the first aspect of the invention.

### Description of the figures

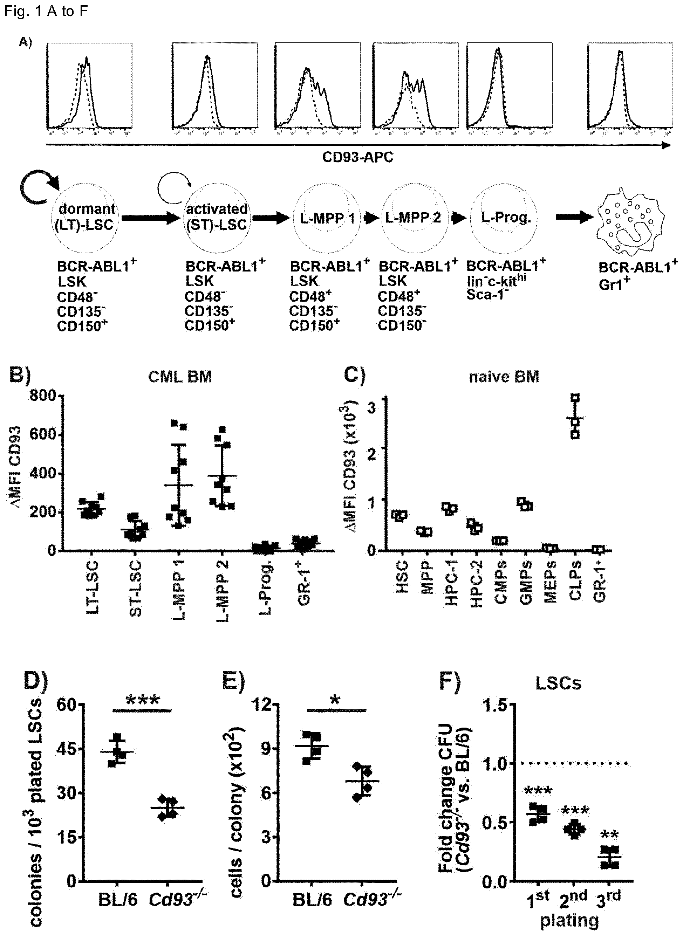

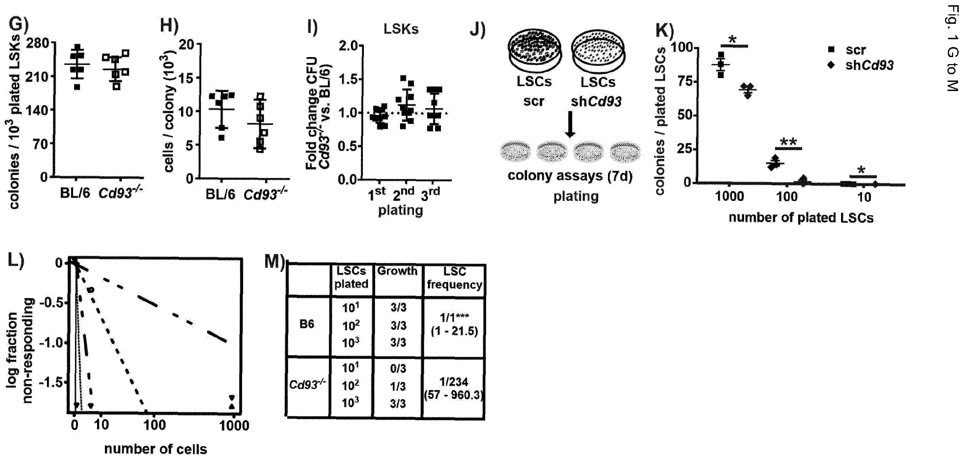

- Fig. 1: shows that CD93-signaling regulates self-renewal of LSCs in vitro. **(A, B)** Representative histograms and mean fluorescence intensities for the expression of CD93 on LSCs, MPPs, leukemic progenitors, and leukemic granulocytes in the BM of CML mice. Isotype is depicted in grey; CD93 staining in black. ΔMFI: MFI staining - MFI isotype (n=9 mice). Pooled data from 2 independent experiments are shown. **(C)** ΔMFI for the expression of CD93 on HSCs, MPPs, HPCs, myeloid and lymphoid progenitors and granulocytes in the BM of naive BL/6 mice. (n=3 mice). One representative out of two experiments is shown. **(D)** Myeloid CFUs per 10³ plated BL/6 and *Cd93*^{*-*/*-*} BM LSCs. **(E)** Colony size. **(F)** Serial replating capacity of BL/6 and *Cd93*^{*-*/*-*} LSCs in vitro (n=4 mice/group). One representative out 2 experiments is shown. **(G)** Myeloid CFUs per 10³ plated BL/6 and *Cd93*^{*-*/*-*} BM LSKs. **(H)** Cells per colony from plated LSKs (n=6 mice/group). One representative out two experiments is shown. **(I)** Serial re-plating capacity of BL/6 and *Cd93*^{*-*/*-*} LSKs in vitro (n=9 mice/group). Pooled data from 2 independent experiments are shown. **(J-M)** Experimental setup. FACS-purified BL/6 LSCs were transduced with sh*Cd93* or scr control RNA particles and positively selected LSCs were plated in methylcellulose at limiting dilution. **(K)** CFUs per 10³ plated sh*Cd93*- or scr vector-transduced LSCs (n=3/group). **(L, M)** ELDA analysis. Data are represented as mean±S.D. Statistics: **(D, E-H, K)** Student's t-test; **(M)** χ2 test; * *p*<0.05, ** *p*<0.01. *** *p*<0.001. Statistically non-significant differences with *p*≥0.05 are not indicated.

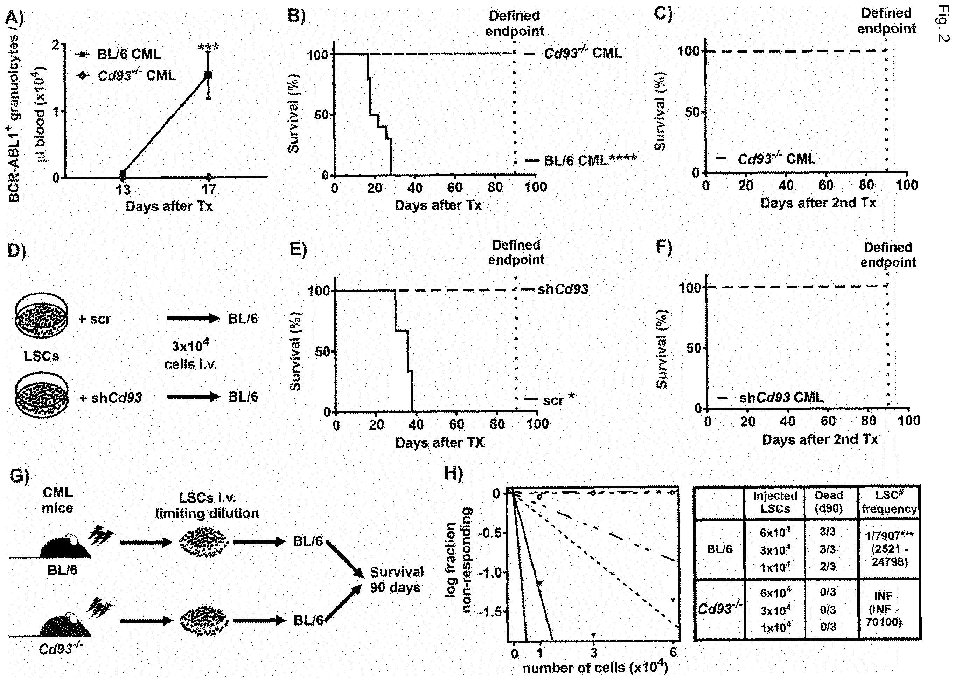

- Fig. 2: shows that CD93-signaling in LSCs promotes CML development. **(A)** Numbers of BCR-ABL1-GFP⁺ granulocytes/µl in blood and **(B)** Kaplan-Meier survival curves resulting from primary transplantations of BCR-ABL1-GFP-transduced BL/6 (BL/6 CML) or *Cd93*^{*-*/*-*} LSKs into naive, non-irradiated BL/6 recipients (n=10 mice/group). Pooled data from 2 independent experiments are shown. **(C)** 5x10⁶ whole BM cells from primary *Cd93*^{*-*/*-*} CML mice 90 days after transplantation were transferred into lethally irradiated (2x 6.5 Gy) secondary recipient mice (n=10) and survival was monitored. **(D-F)** FACS-purified BL/6 LSCs were transduced with sh*Cd93* or scr control RNA lentiviral particles. shRNA-positive LSCs were selected by culture in puromycin-containing medium for 3 days, were then transplanted into naive, non-irradiated BL/6 recipients and survival was monitored. **(D)** Experimental setup. **(E)** Kaplan-Meier survival curves of transplanted mice (n=3 mice per group). **(F)** 5x10⁶ whole BM cells from primary sh*Cd93* CML mice 90 after primary transplantation were injected into lethally irradiated (2x6.5 Gy) recipient mice (n=3), and survival was monitored. One representative experiment out of two independent experiments is shown. **(G, H)** BCR-ABL1-GFP-transduced BL/6 or *Cd93*^{*-*/*-*} LSKs were transplanted into lethally irradiated (2x6.5 Gy) BL/6 recipients. After establishment of the disease, BL/6 or *Cd93*^{*-*/*-*} LSCs were FACS-purified and injected at limiting dilution into naive, non-irradiated BL/6 mice and survival was monitored. **(H)** ELDA analysis. #; LSCs needed to induce CML in immuno-competent hosts. INF = Infinity. One representative experiment out of two independent experiments is shown. Data are represented as mean±S.D. Statistics: **(A)** Two-way ANOVA followed by Bonferroni post-test; **(B, E)** log-rank test; **(H)** χ2 test; * *p*<0.05, ** *p*<0.01. *** *p*<0.001. Statistically non-significant differences with *p*>0.05 are not indicated.

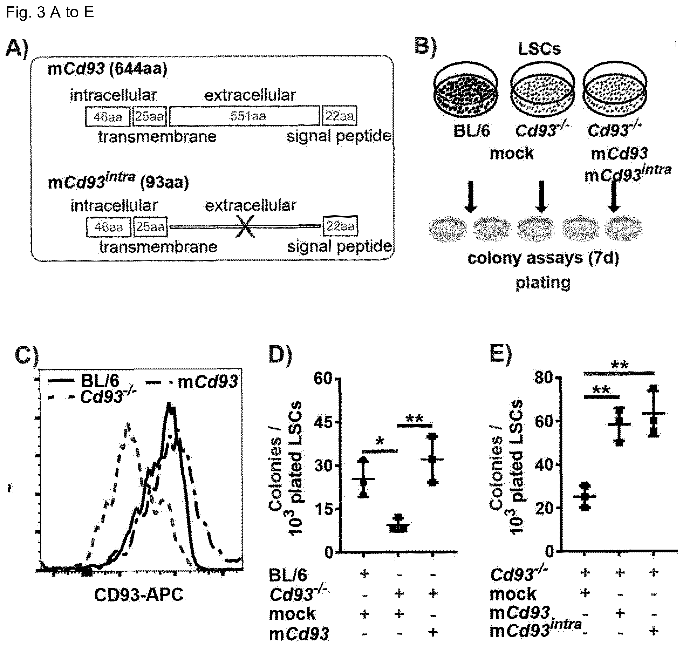

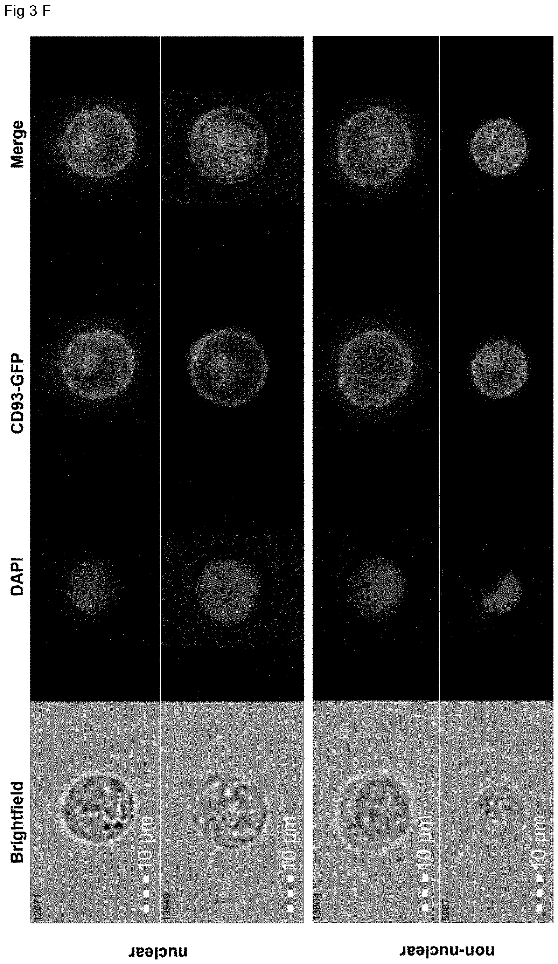

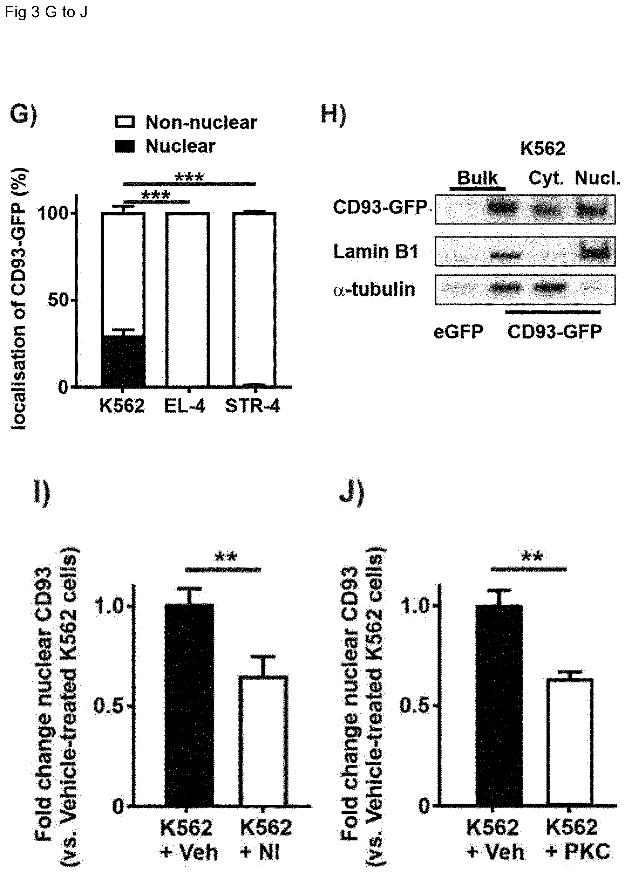

- Fig. 3: shows that CD93-signaling regulates self-renewal of LSCs independent of extracellular ligand-binding. **(A-E)** FACS-purified *Cd93*^{*-*/*-*} CFP⁺ LSCs were transduced with an empty-GFP- (mock) *Cd93*-GFP-expressing (*mCd93*) or *mCd93*^{intra}-GFP-expressing retrovirus. GFP⁺ LSCs were plated in methylcellulose and colony formation was assessed. FACS-purified BL/6 CFP⁺ LSCs transduced with empty-GFP served as control. **(A)** Domain structure of *mCd93* and *mCd9^{intra}.* **(B)** Experimental setup. **(C)** CD93 expression on CFP⁺GFP⁺ LSCs (grey: *Cd93*^{*-*/-} LSCs; black: BL/6 LSC: blue: mCd93 LSCs. **(D)** CFUs per 10³ plated CFP⁺GFP⁺ LSCs (n=3/group). **(E)** Colony formation of FACS-purified *Cd93*^{*-*/*-*} CFP⁺ LSCs transduced with mock, *mCd93* or *mCd93^{intra}* retrovirus. One representative out of two independent experiments is shown. **(F-I)** The BCR-ABL-1 positive cell line K562 and the BCR-ABL-1 negative cell lines EL-4 and STR-4 were transfected with the mammalian expression plasmid pAcGFP1-N1-Cd93 encoding for AcGFP1-N1-Cd93 in triplicates. 48h later cells were analyzed for the sub-cellular localization of CD93. **(F)** Sub-cellular localization of CD93 in K562 cells analyzed by Imagestream. Two representative images of non-nuclear and nuclear CD93-GFP expression are shown. A minimum 1200 GFP⁺ cells pre sample were analyzed. **(G, I)** Percentage of nuclear and non-nuclear CD93-GFP localization analyzed by Imagestream. One representative out of two independent experiments is shown. **(H)** Nuclear and cytosolic CD93-GFP determined by Western Blot. **(I)** Sub-cellular localization of CD93 in K562 cells treated with Nilotinib (NI, 70µM) or vehicle for 72h. One representative out of two independent experiments is shown. **(J)** Sub-cellular localization of CD93 in K562 cells treated with PKC 20-28 (PKC, 50µM) or vehicle for 72h. One representative out of two independent experiments is shown. Data are represented as mean±S.D. Statistics: **(I, J)** Student's t-test; **(D, E, G)** One-way-ANOVA; **(D, E)** Dunnett's post-test (vs. *Cd93*^{*-*/*-*} mock); **(G)** Tukey's post-test. ** *p*<0.01. *** *p*<0.001. Statistically non-significant differences with *p*≥0.05 are not indicated.

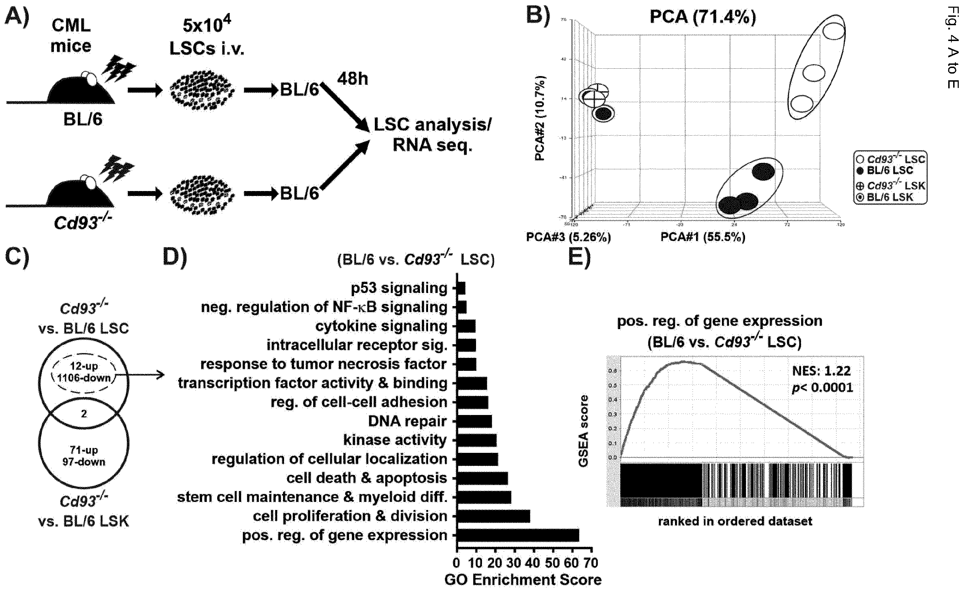

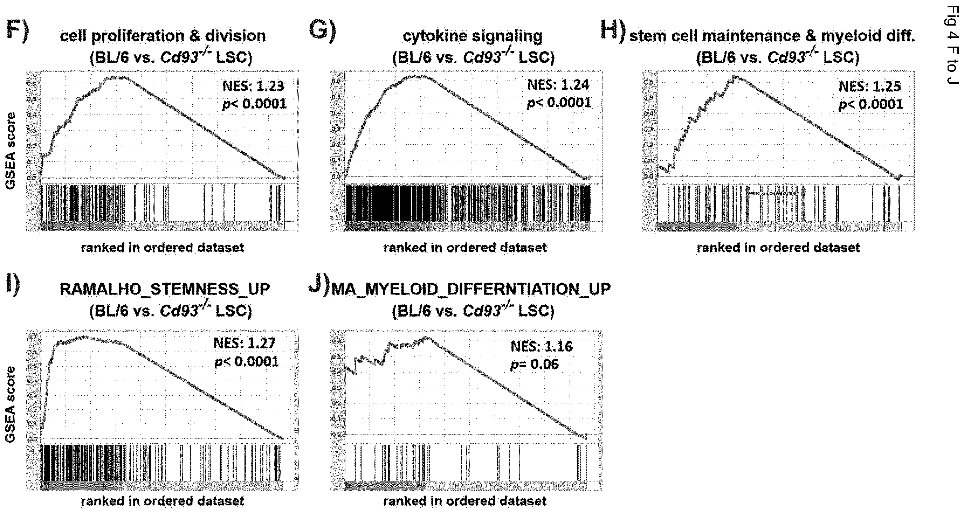

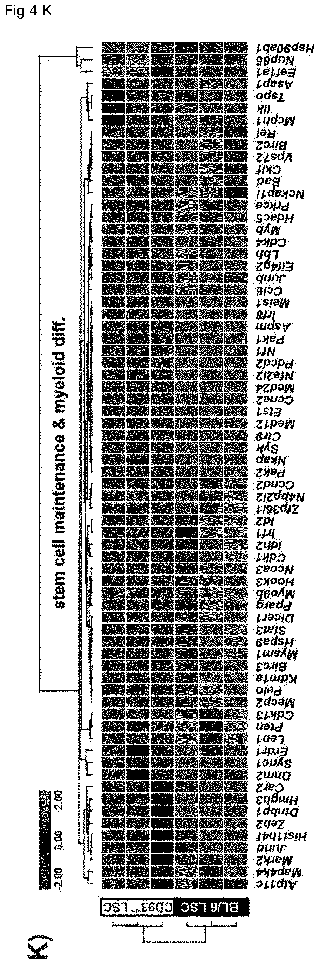

- Fig. 4: shows that CD93 triggers stemness- and proliferation-promoting genes in CML LSCs. **(A)** Experimental Setup. BCR-ABL1-GFP-transduced BL/6 or *Cd93*^{*-*/*-*} LSKs were transplanted into BL/6 lethally irradiated recipients. After establishment of the disease, BL/6 LSCs or *Cd93*^{*-*/*-*} LSCs were purified and 5x10⁴ LSCs were injected into naive BL/6 mice. 48h later LSCs were isolated and gene expression was analyzed by next generation RNA sequencing. **(B)** Principal component analysis (PCA) of BL/6 and *Cd93*^{*-*/*-*} LSKs (n=2 mice/group) as well as BL/6 and *Cd93*^{*-*/*-*} LSCs (n=3 mice per group). **(C)** Venn diagram showing up- and down-regulated genes in *Cd93*^{*-*/*-*} vs. BL/6 LSCs compared to *Cd93*^{*-*/*-*} vs. BL/6 LSKs. **(D)** Gene ontology (GO) analysis (BL/6 vs. *Cd93*^{*-*/*-*} LSCs). A GO enrichment score of ≥ 3 indicates significant change in gene expression. **(E-J)** Gene set enrichment analysis (GSEA) of significantly enriched genes in BL/6 vs. *Cd93*^{*-*/*-*} LSCs. **(K)** Heat map of 70 differentially expressed genes promoting stem cell maintenance and myeloid differentiation. Data are represented as mean ± S.D. Statistics: **(J)** One-way ANOVA. *** *p*<0.001. Statistically non-significant differences with *p*≥0.05 are not indicated.

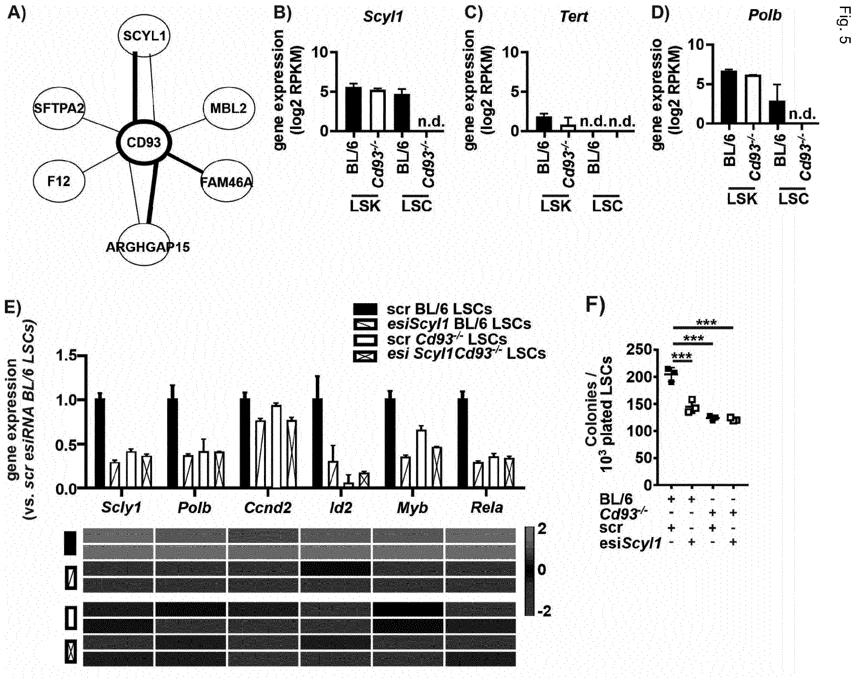

- Fig. 5: shows that Cd93-signaling triggers regulation of gene transcription in via SCYL1 in CML LSCs. **(A)** In silico analysis of predicted CD93 interaction partners using genemania (www.genemania.org). **(B)** *Scyl1,* **(C)** *Tert* and **(D)** *Polb* mRNA expression in *Cd93*^{*-*/*-*} and BL/6 LSKs and LSCs. **(E-F)** FACS-purified BL/6 and *Cd93*^{*-*/*-*} LSCs were transduced with esiScyl1 or scr control esiRNA particles in triplicates and gene expression was determined 48h later(n=3/group). **(E)** Gene expression of *Scyl1, PolB, CCnd2, Id2, Myb, Rela* mRNA expression 48 after gene silencing by esiRNA. **(F)** Colony formation. Data are represented as mean ± S.D. Statistics: One-way-ANOVA followed by Tukey's post-test; *** p<0.001.

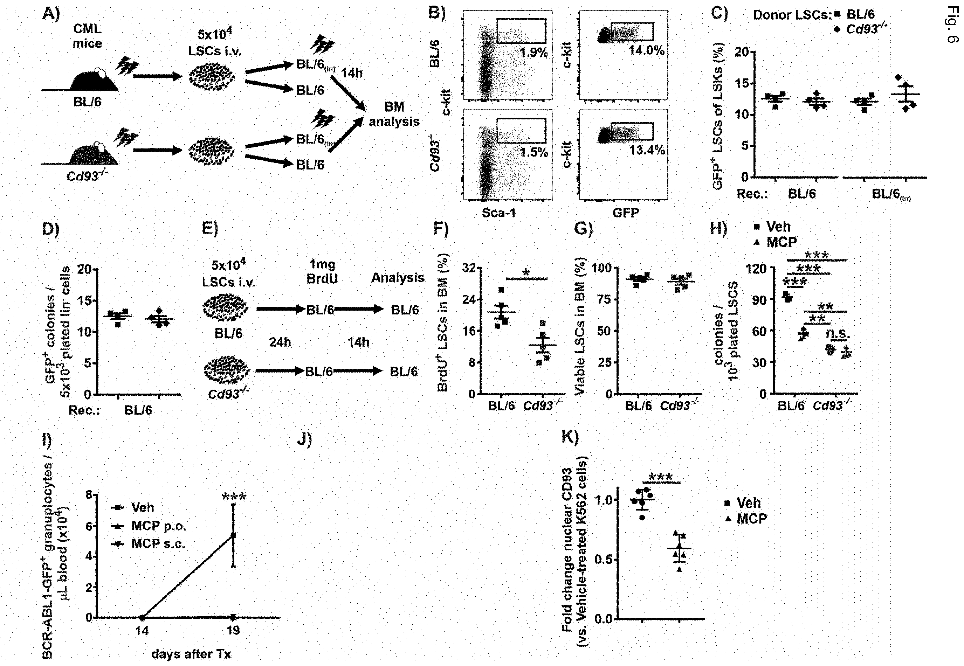

- Fig. 6: shows that CD93 regulates the proliferation of CML LSCs. **(A)** Experimental setup. FACS-purified BL/6 and *Cd93*^{*-*/*-*} LSCs from lethally irradiated (2x 6.5 Gy) primary CML mice were injected i.v. into naive as well as lethally irradiated BL/6 secondary recipient mice. **(B)** Representative FACS plots of BCR-ABL1-GFP⁺BL/6 and *Cd93*^{*-*/*-*} LSCs in the BM of naive BL/6 secondary recipients 14h after transplantation. **(C)** Frequencies of BCR-ABL1-GFP⁺ BL/6 and *Cd93*^{*-*/*-*} LSCs. **(D)** BCR-ABL1-GFP⁺ colony forming unit (CFU) capacity of total lin⁻ cells and For A-D, one representative experiment out of two independent experiments is shown. **(E)** Experimental setup. FACS-purified BL/6 and *Cd93*^{*-*/*-*} LSCs from lethally irradiated primary CML mice were transplanted into naive BL/6 secondary recipients. After 24 h, animals were treated i.p. with 1 mg of BrdU and were sacrificed 14 h later. **(F)** Frequency of BrdU⁺ LSCs. **(G)** LSC viability as determined by Viability dye staining (n=5 mice/group). One representative out of two independent experiments is shown. **(H)** BL/6 and *Cd93*^{*-*/*-*} LSCs were cultured overnight with 1mM metoclopramide followed by plating in methylcellulose. Colony formation was assessed 7 days later. One representative out two independent experiments is shown. **(I)** Numbers of BCR-ABL1-GFP⁺ granulocytes/µl in blood and **(J)** Kaplan-Meier survival curves resulting from primary transplantations of BCR-ABL1-GFP-transduced BL/6 ⁻LSKs into naive, non-irradiated BL/6 recipients. Starting at the day of transplantation, mice were treated with p.o. vehicle or metoclopramide (MCP, 10mg/kg; n=5 mice/group) for 20 days. (K) K562 CML cells were transfected with the mammalian expression plasmid pAcGFP1-N1-Cd93 encoding for AcGFP1-N1-Cd93 in the presence and abscene of 1µM MCP. 72h later cells were analyzed for the sub-cellular localization of CD93 by Imagestream. Pooled data of two independent experiments are shown.
Data are represented as mean ± S.D. Statistics: **(C, D, F, G, K)** Student's t-test; **(H)** One-way ANOVA followed by Tukey's post-test; **(I)** Two-way ANOVA followed by Bonferroni post-test; **(J)** log-rank test;* *p*<0.05. Statistically non-significant differences with p≥0.05 are not indicated.

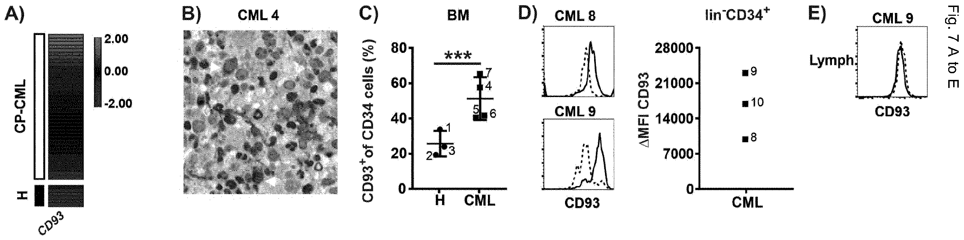

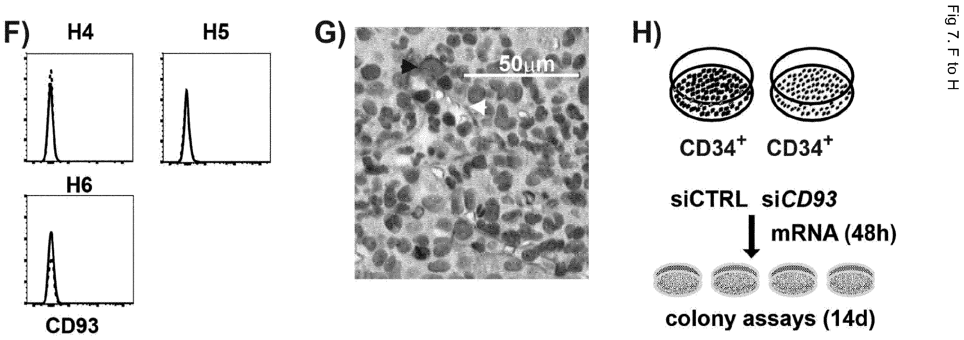

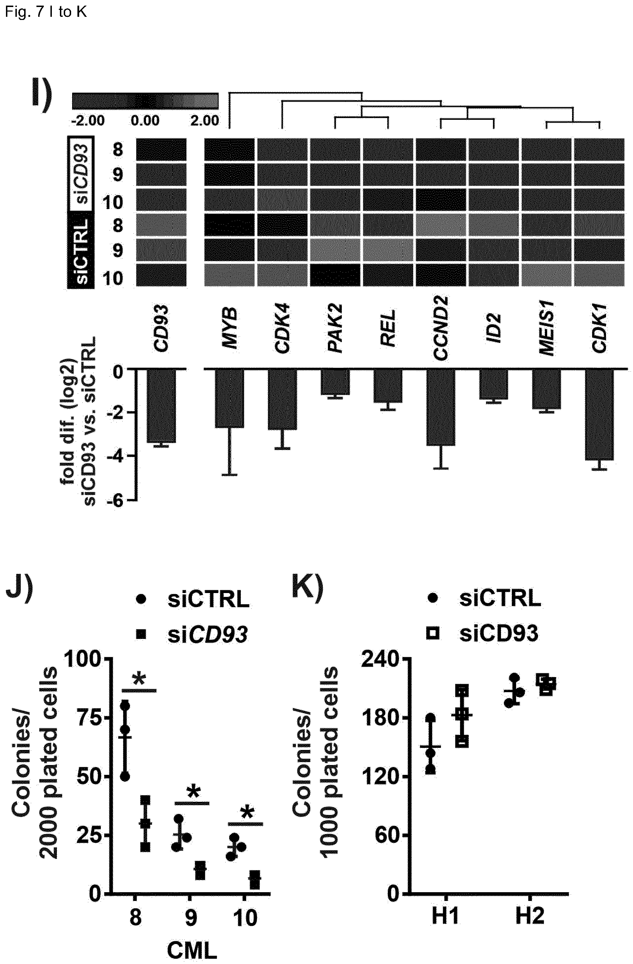

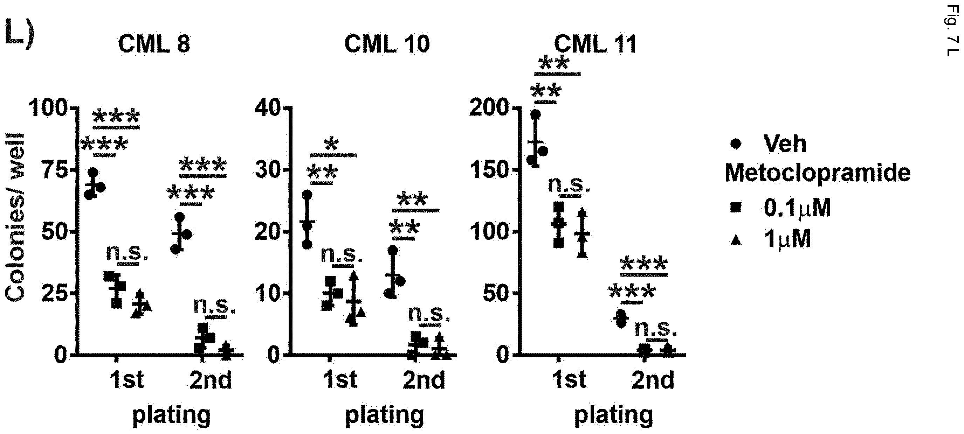

- Fig. 7: shows that CD93 regulates colony formation capacity of CD34⁺ BM stem/progenitor cells from CML patients in vitro. **(A)** A publicly available microarray dataset (GSE4170) that assessed gene expression profiles in CML was analyzed for CD93 using the Gene Expression Omnibus GEO2R tool. Expression values of patients in chronic-phase CML (n=42; green bars) and CD34⁺ stem/progenitor cells from healthy donor BM (n=5; black bars) are shown. **(B-C)** CD93⁺CD34⁺ stem/progenitor cells in the BM of healthy donors (n=3) and untreated CML patients (n=4) were determined by immunohistochemistry (IHC) and cell morphology. **(B)** Representative IHC image. CD93 (brown), CD34 (red). Scale bar: 20µm. White arrows indicate CD93⁺CD34⁺ double-positive stem/progenitor cells. 145-328 CD34⁺ BM cells were analyzed per trephine biopsy. **(C)** Percentage of CD93-expressing CD34⁺ cells in CML BM as analyzed by IHC. **(D)** Representative FACS histograms (left panel) and the mean fluorescence intensity (MFI, right panel) of CD93 expression vs. isotype control on lin⁻ CD34⁺ BM cells from CML patients. ΔMFI: MFI staining - MFI isotype. **(E)** CD93 expression on lymphocytes (CD45^{hi}SSC^{lo}) in human CML BM as analyzed by FACS. One representative histogram of n=3 is shown (CML patients 8-10). **(F)** Histograms of CD93 expression on CD34⁺ BM stem/progenitor cells from healthy donors (H4-H6) as analyzed by FACS. Black lines, CD93 staining; grey lines, isotype controls. **(G)** Representative IHC image of CD34 and MMRN2 co-stainings in the BM of healthy donors (n=3) and untreated CML patients (n=4). MMNR2 (brown), CD34 (red). Scale bar: 50µm. **(H-J)** lin⁻CD34⁺ BM stem/progenitor cells from CML patients 8-10 were treated with si*CD93* RNA (si*CD93*) or control siRNA (siCTRL) for 48 h in triplicates, followed by plating in methylcellulose or RNA isolation. **(H)** Experimental setup. **(I)** Heat map for the expression of *CD93* and selected genes involved in self-renewal and cell proliferation in siCTRL vs. si*CD93* cells. **(J, K)** Colony formation per 2000 plated **(J)** leukemia- and **(K)** 1000 normal stem and progenitor cells. **(L)** CD34⁺ BM CML stem/progenitor cells (CML 8, 10 and 11) were treated with titrated concentrations of metoclopramide or vehicle overnight in triplicates, followed by plating in methylcellulose. Colony formation of 2000 plated cells for primary platings in shown. For secondary platings, 2x10⁴ cells isolated from primary colony assays were re-plated in methylcellulose in the absence of the compound. Data are represented as mean ± S.D. Statistics: **(C, J, K)** Student's t-test; **(L)** One-way ANOVA followed by Tukey's post-test. * *p*<0.05, ** *p*<0.01, *** *p*<0.001. Statistically non-significant differences with *p*≥0.05 are not indicated.
- Fig. 8: shows promethazine HCl.
- Fig. 9: shows metoclopramide HCl.
- Fig. 10: shows chlorpromazine HCl.

### Examples

The inventors found CD93 expression on LSCs and leukemia progenitor cells but not on more differentiated leukemia granulocytes in a murine retroviral lineage-negative Sca-1⁺ c-kit⁺ (LSK) transduction/transplantation CML model. Next-generation sequencing analysis revealed that *Cd93*^{*-*/*-*} LSCs have a silenced gene expression signature particularly in genes involved in the regulation of gene expression, stem cell maintenance and proliferation. Out of the 1120 genes differentially expressed between BL/6 and *Cd93*^{*-*/*-*} LSCs, 1108 genes were down-regulated. In contrast, naive BL/6 and *Cd93*^{*-*/*-*} hematopoietic stem cells (HSCs) did not display a dysregulation in these pathways. Functionally, CD93-deficiency in LSCs resulted in impaired self-renewal, reduced LSC frequencies in vitro (at least by a factor of 100, *P*<0.001) and in the incompetence to induce and propagate CML in mice.

To study whether CD93-signaling in LSCs relies on ligand-binding to the extracellular domain of CD93, an extracellular domain deletion mutant of CD93 (m*Cd93*^{intra}) was generated. Comparable to transduction with full-length m*Cd93*, the expression of Cd93^{intra} restored colony formation of *Cd93*^{*-*/*-*} LSCs in vitro, suggesting that the maintenance of LSC self-renewal is independent of ligand-binding to the extracellular domain of CD93. Furthermore, analysis of the sub-cellular localization of CD93 in CML cells using a lentiviral expression vector encoding for AcGFP1-N1-*Cd93* demonstrated nuclear localization of the CD93 intracellular domain (ICD). SCY1 like pseudokinase 1 (SCYL1), a regulator of gene transcription, directly interacts with the highly charged juxta membrane domain of the cytoplasmic tail of CD93 (Bohlson et al, 2005). Silencing of Scyl1 significantly reduced colony formation of BL/6 but not Cd93^{-/-} LSCs in vitro suggesting that the ICD of CD93 regulates gene transcription via Scyl1 in CML LSCs.

To discover compounds that affect LSC function similarly as genetic CD93 blockade, the inventors performed a compound screen. The antiemetic agent metoclopramide, which is widely used in clinical routine to reduce nausea in cancer patients, was one very promising candidate identified in the screen. Metoclopramide treatment reduced clonogenic potential of CD93-competent LSCs to comparable levels as CD93-deficient LSCs in vitro without further affecting colony formation of CD93-deficient LSCs.

Analysis of LSCs from newly diagnosed CML patients similarly demonstrated that CD93-signaling induces the expression of genes associated with proliferation and stemness, resulting in an increased clonogenic potential in vitro. In addition, colony formation and re-plating capacity in semisolid cultures of human CD34⁺CD38⁻ LSCs was significantly impaired by metoclopramide at a pharmacological concentration of 0.1µM compared to control treatment.

Overall, these results indicate that CD93-siganling is an important regulator of stemness and proliferation of human and murine CML LSCs. Furthermore, this study identifies expression of CD93 by LSCs as promising novel target for the treatment of CML.

### Materials and methods

### Study design

In hypothesis-driven experimental designs, the molecular mechanisms of *Cd93*-signaling in LSCs in a murine model of CML using BL/6 and *Cd93*^{*-*/*-*} mice were addressed. In addition, leukemia development, survival, LSC proliferation and LSC activity was assessed. All experiments were performed with 6- to 8-week-old mice housed in a specific pathogen-free facility in individually ventilated cages. Food was provided ad libitum. Mice were assigned randomly to different treatment groups. These investigations were extended to CML cell lines and primary BM samples from CML patients to further investigate the molecular mechanisms and demonstrate the human relevance of the findings.

All experiments were conducted and analyzed in a non-blinded fashion. Experiments were performed one to two times. Details on replicates are indicated in figure legends.

### Antibodies

Mouse: αCD34-eFluor 450 (clone RAM34), αc-kit-PE-Cy7 and -APC-Alexa750 (clone 2B8), αSca-1-PerCP-Cy5.5 (clone D7), αCD16/32-PE-Cy7 (clone 93), αCD90-AlexaFluor 700 (clone 30-H12), αCD150-Pacific blue (clone TC15-12.2), αCD127-PE (clone A7R34), αCD48-AlexaFluor 700 (clone HM48-1), αGr-1-PE and -biotin (clone RB6-8C5), CD135-PE (clone A2F10), αCD19-APC-Cy7 and -biotin (clone 6D5), CD11b-PE-Cy7 (clone M1/70), αCD3α-biotin (clone 145-2C11) and αTer119-biotin (clone Ter-119) were from BioLegend. αCD93-APC (clone AA4.1) and the corresponding isotype control (clone RTK453) were from eBioscience. Streptavidin-BDHorizonV500 was from BD Pharmingen. Lineage depletion was performed with a cocktail of lineage-specific antibodies (αCD3α-biotin, αCD19-biotin, αGr-1-biotin, αTer119-biotin) using αbiotin microbeads and autoMACS system (Miltenyi Biotec).

Human: αCD34-APC and -APC-Cy7 (clone 561), αCD45-Pacific-Blue (clone HI30), αCD38-PE-Cy7 (clone HIT2) and αCD90-PerCP-Cy5.5 (clone 5E10) were from BioLegend. Lineage-positive cells were excluded by staining using biotinylated αCD2 (clone RPA2.10), αCD3 (clone OKT39), αCD14 (clone HCD14), αCD16 (clone 3G8), αCD19 (clone HIB19), αCD56 (clone HCD56) and αCD235 (clone HIR2) (BioLegend), followed by a second step using streptavidin-BD-Horizon-V500 (BD Pharmingen). αCD93 (clone HPA009300) was from Millipore Sigma. Goat-anti-rabbit Alexa647 secondary antibody was from ThermoFisher. Fixable viability dye-eFluor450 was from eBioscience.

Samples were acquired on a BD LSRFortessa and sorting procedures were conducted using a BD FACS Aria III (BD Biosciences). Data were analyzed using FlowJo software (TreeStar).

### Patient samples

BM aspirates from untreated, newly diagnosed CML patients at the Department of Hematology and Central Hematology Laboratory, Inselspital, Bern University Hospital and University of Bern, Switzerland, were obtained after written informed consent. Patient characteristics are listed in table S3. Analysis of BM samples was approved by the local ethical committee of the Canton of Bern, Switzerland (KEK 122/14).

### In vitro experiments

### Cell lines

The leukemia cell line BCR-ABL1-expressing leukemia cell lines K562 (Chen et al, 1985) and EL-4 (Gorer et al, 1950) was purchased from ATCC. No additional authentication was performed. The BM endothelial cell line STR-4 (Aizawa et al, 1991) was kindly provided by Prof. M. Manz (University Hospital Zürich, Switzerland). The cells line were tested mycoplasma-free and was were grown in FCS-containing medium recommended by ATCC (https://www.lgcstandards-atcc.org/?geo_country=ch) supplemented with 100U/ml penicillin, and 100µg/ml of streptomycin in a humidified atmosphere of 95% air and 5% CO2 at 37°C at a low passage number. Media were routinely changed every 3 days (Chen et al, 1985.

### Colony assays

Colony assays from BM lin⁻ cells of CML and naive mice and CD34⁺ stem/progenitor cells of human CML patients were performed as described before (Riether et al. 2015).

For mouse colony assays 10³ FACS-purified LSKs or LSCs were plated in methylcellulose and colonies were enumerated 7 days later. For serial re-plating experiments, 10⁴ cells were collected from preceding colony assays and re-plated in methylcellulose. Colonies were enumerated 7 days later.

For experiments using metoclopramide (Sigma), 10³ FACS-purified murine LSCs or 2x10³ human CD34⁺ stem/progenitor cells were incubated overnight in 96 well V-bottom plates in the presence of 0.1 and 1µM metoclopramide or vehicle followed by plating methylcellulose. Colony were enumerated 7 days and 14 days later, respectively.

For re-plating assays 10⁴or 2x10⁴ cells isolated from primary mouse and human colony assays, respectively, were subsequently replated in methylcellulose in the absence of any compound. Colony were enumerated 7 days and 14 days later, respectively.

### Gene silencing of CD93, Cd93 and Scyl1.

siRNA: Human CD93 and murine *Scyl1* was silenced in human lin⁻CD90⁺CD34⁺ BM cells and murine LSCs, respectively, using siRNA and esiRNA for gene silencing according to the manufacturer's instructions (Santa Cruz Biotechnology and Sigma, respectively) with minor modifications to increase silencing efficiency. Briefly, siRNA, esiRNA or scrambled controls were mixed with transfection medium and Lipofectamine LTX (Invitrogen) in serum-free transfection media. Cells were subsequently treated with transfection complexes in the presence of antibiotic- and FBS-free growth medium overnight and were further used for functional assays and determination of the expression for selected genes of interest.

shRNA: *Cd93* mRNA was silenced in murine LSCs using ready-to-use lentiviral particles (Santa Cruz Biotechnology). Briefly, 5x10⁴ LSCs cells were transduced overnight by spin-transfection at 37°C and 5% CO₂ with virus of sh*Cd93* lentiviral particles or the respective control scrambled RNA lentiviral particles (MOI: 5, Santa Cruz Biotechnology Inc.) in the presence of 5µg/ml polybrene (Millipore, Sigma). After spin transfection, medium was removed and cells were cultured in medium supplemented with 1µg/ml puromycin for 48h to select for stable expression of sh*Cd93* or scrambled (scr) RNA.

### Overexpression of mCd93.

The mouse *Cd93* gene (ENSMUSG00000027435; *mCd93*) was cloned from FACS-purified B cell precursors. cDNA synthesis was carried out using SuperScript III Reverse Transcriptase kit (Thermo Fisher Scientific). The open reading frame (ORF) of *Cd93* with a total length of 1.932kb (644aa) was amplified by PCR using tagged primes for the restriction enzymes (EcoRI and Xhol). In addition, the forward primer harbored the Kozak consensus sequence (GCCACC) before the start codon. Next, the Cd93 ORF was cloned into pMSCV-IRES-GFP II (pMIG II) plasmid (Addgene: #52107).

The *Cd93^{intra}* construct, lacking the sequence encoding for the extracellular domain of *Cd93* was designed by cloning of the signal peptide (1-22aa), transmembrane (574-598aa) and cytoplasmic (599-644aa) domains of the *Cd93* ORF (total length of 282bp) into a pMIG II plasmid. To predict and select the different Cd93 protein domains prior to cloning, the Phobius database (phobius.sbc.su.se) was used.

Retroviral vectors expressing *Cd93* or *Cd93^{intra}* were generated using Platinum-E (Plat-E) Packaging Cell Line (Cell Biolabs). Next, FACS-purified primary CML LSCs derived from *Cd93*^{*-*/*-*} mice were transduced with retroviral vectors expressing *Cd93, Cd93intra* or an empty vector as a control (mock). Two days after transduction, LSCs expressing *Cd93-, Cd93^{intra}* or mock GFP were FACS-purified and plated in methylcellulose.

### Sub-cellular localization of mCd93.

The mouse Cd93 gene was cloned in pAcGFP1-N1 mammalian expressing plasmid and as the lentiviral construct pLVX-AcGFP1-N1 (Clontech). Lentiviral particles were generated in 2the 93FT packaging cell line (Invitrogen) using pMDLg/pRRE, pRSV-Rev and phCMV-VSV-G 3rd generation plasmids. For transfection, adherent STR-4 cells were seeded at density 10⁵ cells/well in antibiotics-free medium and left to adhere for 5h. Cells growing in suspension (K562 and EL4 cells) were seeded at a density 10⁵ cells/well antibiotics-free medium shortly before transfection. Liposomal transfection was performed with Lipofectamine LTX according to the manufacturers' instructions (Thermo Fisher Scientific), using the previously optimized conditions (per 10⁵ cells: 0.25µg plasmid DNA, 2.5µL Lipofectamine LTX, and for K562 additionally 0.25µL PLUS reagent). 48h after transfection/transduction, the expression of mCD93-GFP fusion protein was analyzed by ImageStream or Western Blot. For experiments with Nilotinib, 70µM of Nilotinib or vehicle (DMSO) were added to the culture for 72h. For experiments using the PKC-α/β inhibitor PKC 20-28 (Merck) or metoclopramide (Sigma), 50µM and 0.1µM of compound or vehicle (H₂0) were added to the culture for 72h, respectively.

### ImageStream data acquisition and analysis.

Briefly, cells were stained with fixable viability dye eFluor780 (Thermo Fisher Scientific) for 30 min at 4°C. Cells were subsequently fixed with 4% paraformaldehyde for 10 min at room temperature and then permeabilized for 5 min at room temperature with DAKO Wash (Dako) diluted in DAKO diluent (Dako). After washing, nuclei were stained with 10 µg/ml DAPI or NucRed Live 647 ReadyProbes (Thermo Fisher Scientific) according to the manufacturers' instructions. Data was acquired on the Amnis ImageStreamX Mark II instrument. Spectral compensation, background correction and adaption of the nuclear mask was performed, and images were analyzed with the IDEAS image analysis software by calculating nuclear translocation of GFP-labelled CD93. A minimum 1200 GFP⁺ cells were analyzed per sample.

### Sub-cellular fractionation and Western Blot.

After the FACS-sorting, K562 cells expressing pLVX-AcGFP1-N1 or pLVX-AcGFP1-N1-*Cd93* were washed twice with ice-cold PBS. The cell pellet was re-suspended in 0.1% NP40 (Thermo Scientifi 28324)-PBS. An aliquot was removed as the whole cell lysate. The remaining lysate was centrifuged for 10 sec. and the supernatant was transferred to a new tube as the cytoplasmic fraction. The pellet was re-suspended in ice-cold NP40-PBS followed by the centrifugation. The supernatant was discarded, and the remaining pellet was kept as a nuclear fraction. The whole cell lysate and the nuclear fraction were re-suspended in 1x Laemmli buffer (Biorad) containing β-Mercaptoethanol followed by sonication using microprobes 5x for 2 sec. at 40% intensity. The cytoplasmic fraction was resuspended in Laemmli buffer; all samples were boiled for 5min and loaded on the 4-20% Mini-PROTEAN® TGX Stain-Free™ Protein Gels (Biorad). The transfer was performed using Trans-Blot® Turbo™ RTA Mini PVDF Transfer Kit (Biorad). The membranes were blocked for 2h in 5% BSA and incubated overnight, at 4°C, with the following antibodies: αGFP (abcam), α-Lamin B1 (Abcam) and α-alpha Tubulin (DM1A) (Abcam). The membranes were incubated with HRP conjugated secondary antibodies: anti-mouse IgG (Cell signaling) or anti-rabbit IgG (Cell signaling). Proteins were detected with Clarity™ Western ECL Substrate (Biorad) and visualized on ChemiDoc™ Imaging Systems.

### Immunohistochemistry (IHC)

IHC stainings were performed on a Leica BOND RX automated immunostainer (Leica Biosystems). Thin sections (1-2 µm) of FFPE tissue were pre-treated by boiling at 100°C in citrate buffer, pH 6.0 for 30 min. Double stainings were performed sequentially: First, slides were stained with rabbit anti-human CD93 (polyclonal, Millipore Sigma #HPA012368; dilution 1:50) for 30 min, followed by visualization using the Bond Polymer Refine DAB Detection kit (Leica Biosystems). Then, slides were counterstained using mouse anti-human CD34 (clone QBEnd/10, Cell Marque #134M-16; dilution 1:200) for 15 min, followed by visualization using the Bond Polymer Refine Red Detection kit (DS9390, Leica Biosystems). Finally, the samples were counterstained with hematoxylin and mounted with Aquatex® (Millipore Sigma). Slides were digitized using a Pannoramic 250 Flash III scanner (3DHistech). Analysis and quantification of the stainings was performed under the supervision of a board-certified surgical pathologist (C.M.S.).

### High-throughput transcriptome analysis using next generation RNA sequencing (NGS)

Total RNA was extracted from naive BL/6 and CD93^{-/-} LSKs (n=2/group) and LSCs (n=3/group) using the RNeasy Micro Kit (Qiagen). Total RNA quality was determined by a Bioanalyzer using the RNA 6000 Nano Chip (Agilent Technologies) and quantified by fluorometry using the Quantifluor RNA System Kit on a Quantus Fluorometer Instrument (Promega).

Library preparation was performed from total RNA using the SMART-Seq v4 Ultra Low Input RNA Kit for Sequencing (Takara Bio). Libraries were quality-checked on the Fragment Analyzer using the High Sensitivity NGS Fragment Analysis Kit (Advanced Analytical). Samples were pooled to equal molarity and the pool was quantified by fluorometry, in order to be loaded at a final concentration of 2pM on the NextSeq 500 instrument (Illumina). Samples were sequenced SR76 using the NextSeq 500 High Output Kit 75-cycles (Illumina) and primary data analysis was performed using the Illumina RTA version 2.4.11 and bcl2fastq v2.20.0.422.

### NGS data analysis

The NGS data was assembled by SeqMan NGen software v.15 and analyzed using ArrayStar software v.15 (DNASTAR, USA). The level of gene expression was assessed after reads per kilobase of target per million reads mapped (RPKM) normalization and log2 transformation. The data set was analyzed by two-way ANOVA. Genes with significant difference in their expression at FDR-p<0.05 and fold differences ≥ 1.5 were selected. Data were clustered using standard Euclidean's method based on the average linkage and heatmaps were generated according to the standard normal distribution of the values.

### Gene ontology and gene set enrichment analysis.

Gene ontology (GO) enrichment was assessed using Partek® Genomics Suite™ software, v.7 (Partek). The list of significantly differently expressed genes was grouped into functional hierarchies. Enrichment scores were calculated using a chi-square test comparing the proportion of the gene list in a group to the proportion of the background genes. A value of 3 or higher corresponded to a significant over-expression (p<0.05). Gene set enrichment analysis (GSEA) was performed using GSEA software v.3.0 (Broad Institute). Enrichment analysis was assessed for all pathway-related genes acquired from pathcards database (pathcards.genecards.org) and the gene sets RAMALHO_STEMNESS_UP (Ramalho-Santos et al . 2002) and MA_MYELOID_DIFFERENTIATION_UP (Husain et al. 2002).

### Gene expression profiling of human CML CD34⁺ LSCs upon CD93 knock-down.

Total RNA was extracted from FACS-sorted CD34⁺ LSCs of three human CML patients after 48h treatment with siCD93 or siCtrl using the RNeasy Micro Kit (Qiagen). For each sample, cDNA synthesis was assessed using High Capacity cDNA Reverse Transcription Kit (Applied Biosystems, USA). Primers were designed for each gene using Primerquest Software (Integrated DNA Technologies) (Table S5). For qRT-PCR analysis, synthesized cDNAs amplified with specific primers using FastStart Universal SYBR® Green 2X PCR Master Mix (Roche, Switzerland). Raw values were normalized using geometric mean of reference genes (ACTB and GAPDH). Real-time PCR reactions were performed in two replicates and included no-template controls using ABI Prism 7500 Sequence Detection System (Applied Biosystems). The fold difference for each sample was calculated using the comparative Ct method.

### Animal experiments

Animal experiments were approved by the local experimental animal committee of the Canton of Bern and performed according to Swiss laws for animal protection.

### Mice

C57BL/6J (BL/6) mice were from Charles Rivers (Sulzfeld, Germany). *Cd93*^{*-*/*-*} on BL/6 background were provided by Prof. Hans Acha-Orbea (University of Lausanne). BL/6 background was confirmed by SNP analysis performed at Taconic.

### CML model.

FACS-purified LSKs were harvested and transduced twice with BCR-ABL1-GFP or -CFP retrovirus by spin infection at a multiplicity of infection (MOI) of 0.5. 3x10⁴ cells were injected intravenously (i.v.) into the tail vein of non-irradiated syngeneic recipients. To determine residual disease in surviving mice, CML mice were sacrificed 90 days after transplantation and 5x10⁶ BM cells were injected i.v. into lethally irradiated (2x 6.5 Gy) recipient mice.

For MCP treatment, CML mice were treated daily once p.o. or s.c. with MCP (Sigma) at a concentration of 10mg/kg (Shalaby et al. 2013) starting at the day of CML induction.

### LSPC and HSPC analysis.

LSCP numbers in CML mice an HSCP numbers in naive mice were analyzed as described before (Riether et al, 2015, Schurch et al 2015).

### LSC homing experiments.

5x10⁴ BL/6 or *Cd93*^{*-*/*-*} LSCs were transplanted i.v. into lethally irradiated (2x 6.5Gy) or non-irradiated BL/6 mice. 14h later mice were sacrificed and BM was analyzed for the presence of GFP⁺ LSCs.

### BrdU incorporation in vivo.

CML mice were injected i.p. with 1 mg BrdU. 14 h later, mice were sacrificed and BrdU staining was performed using the BD Pharmingen BrdU Flow Kit according to the manufacturer's instructions.

### Statistical analysis

Statistical analysis was performed using GraphPad Prism® software v7.0 (GraphPad). Bars and error bars indicate means and standard deviations, respectively, if not otherwise specified. Data were analyzed using Student's t-test, one-way-ANOVA followed by Dunnett's or Tukey's post-test, two-way ANOVA followed by Bonferroni post-test and log-rank test. LSC frequencies with 95% confidence intervals (CI) were estimated with ELDA software (http://bioinf.wehi.edu.au/software/elda/) and significant differences in LSC frequency were calculated by χ2 test in limiting dilution assays according to (Hu et al. 2009). Significance of differences in Kaplan-Meier survival curves was determined using the log-rank test (two-tailed). p<0.05 was considered significant.

### References

T. L. Holyoake, D. Vetrie, The chronic myeloid leukemia stem cell: stemming the tide of persistence, Blood 129, 1595-1606 (2017).
R. Kinstrie, G. A. Horne, H. Morrison, H. A. Moka, J. Cassels, K. Dunn, P. Herzyk, D. A. Irvine, M. Copland, CD93 Is a Novel Biomarker of Leukemia Stem Cells in Chronic Myeloid Leukemia, Blood 126 (2015).
C. Riether, C. M. Schurch, C. Flury, M. Hinterbrandner, L. Druck, A. L. Huguenin, G. M. Baerlocher, R. Radpour, A. F. Ochsenbein, Tyrosine kinase inhibitor-induced CD70 expression mediates drug resistance in leukemia stem cells by activating Wnt signaling, Sci. Transl. Med. 7, 298ra119 (2015).
C. Riether, T. Gschwend, A. L. Huguenin, C. M. Schurch, A. F. Ochsenbein, Blocking programmed cell death 1 in combination with adoptive cytotoxic T-cell transfer eradicates chronic myelogenous leukemia stem cells, Leukemia 29, 1781-1785 (2015).
T. R. Chen, Modal karyotype of human leukemia cell line, K562 (ATCC CCL 243), Cancer Genet. Cytogenet. (1985), doi:10.1016/0165-4608(85)90101-3.
P. A. Gorer, Studies in antibody response of mice to tumour inoculation, Br. J. Cancer (1950), doi:10.1038/bjc. 1950.36.
S. Aizawa, M. Yaguchi, M. Nakano, S. Inokuchi, H. Handa, K. Toyama, Establishment of a variety of human bone marrow stromal cell lines by the recombinant SV40-adenovirus vector, J. Cell. Physiol. (1991), doi:10.1002/jcp.1041480209.
M. Ramalho-Santos, S. Yoon, Y. Matsuzaki, R. C. Mulligan, D. A. Melton, "Stemness": Transcriptional profiling of embryonic and adult stem cells, Science (80-.). (2002), doi:10.1126/science.1072530.
X. Ma, T. Husain, H. Peng, S. Lin, O. Mironenko, N. Maun, S. Johnson, D. Tuck, N. Berliner, D. S. Krause, A. S. Perkins, Development of a murine hematopoietic progenitor complementary DNA microarray using a subtracted complementary DNA library, Blood (2002), doi:10.1182/blood.V100.3.833.
C. M. Schurch, C. Riether, A. F. Ochsenbein, Cytotoxic CD8+ T cells stimulate hematopoietic progenitors by promoting cytokine release from bone marrow mesenchymal stromal cells, Cell Stem Cell 14, 460-472 (2014).
Y. Hu, G. K. Smyth, ELDA: Extreme limiting dilution analysis for comparing depleted and enriched populations in stem cell and other assays, J. Immunol. Methods 347, 70-78 (2009).
M. A. F. Shalaby, Interaction of insulin with prokinetic drugs in STZ-induced diabetic mice, World J. Gastrointest. Pharmacol. Ther. (2013), doi:10.4292/wjgpt.v4.i2.28.

## Claims

1. A compound of formula 1 for use in the treatment of cancer,
Ar-L-N(R¹)(R²) (1),
wherein
Ar is a mono-, bi or tricyclic aryl or heteroaryl, wherein Ar is unsubstituted or substituted with one or more substituents R³, wherein
R³ is independently selected from
-C₁₋₆-alkyl, -C₂₋₆-alkenyl, -C₂₋₆-alkynyl,
-F, -Cl, -Br, -I, -CN,
-NR^{a}₂, -C₁₋₆-alkyl-NR^{a}₂,
-OR^{a}, -C₁₋₆-alkyl-OR^{a}
-SR^{a}, -C₁₋₆-alkyl-SR^{a},
with each R^{a} being selected independently from each other from H, a C₁₋₆ alkyl, a C₂₋₆ alkenyl, or a C₂₋₆ alkynyl,
wherein the -C₁₋₆-alkyl, -C₂₋₆-alkenyl or -C₂₋₆-alkynyl is unsubstituted or halogen substituted, particularly unsubstituted or substituted with -F,
L is a linker,
N is a nitrogen atom,
R¹ and R² are independently selected from C₁₋₆-alkyl, or
R¹ and R² form a saturated aliphatic or heterocyclic ring.

2. The compound for use in the treatment of cancer according to claim 1, wherein R¹ and R² are independently selected from C₁₋₄-alkyl, particularly from methyl and ethyl.

3. The compound for use in the treatment of cancer according to any one of claims 1 or 2, wherein the mono- bi- or tricyclic aryl or heteroaryl comprises one, two or three 5- or 6-membered rings.

4. The compound for use in the treatment of cancer according to any one of the preceding claims, wherein the 5- or 6-membered rings of the bi- or tricyclic aryl or heteroaryl are fused.

5. The compound for use in the treatment of cancer according to any one of the preceding claims, wherein Ar is a moiety of formula 2 or 3, 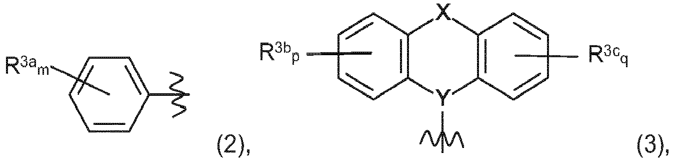 wherein
X is selected from -CH-, -NH-, -O- and -S-, particularly -O- and -S-, more particularly - S-,
Y is -N- or -CH-, particularly -N-,
R^{3a}, R^{3b} and R^{3c} are independently selected as described above for R³,
m is 0, 1, 2, 3, 4 or 5, particularly 0, 1, 2 or 3, more particularly 1, 2 or 3,
p is 0, 1, 2, 3, 4, particularly 0, 1 or 2, more particularly 0 or 1,
q is 0, 1, 2, 3, 4, particularly 0, 1 or 2, more particularly 0 or 1.

6. The compound for use in the treatment of cancer according to claim 5, wherein R^{3a}, R^{3b} and R^{3c} are independently selected from
-C₁₋₄-alkyl, -C₂₋₄-alkenyl, -C₂₋₄-alkynyl,
-F, -Cl, -Br, -I, -CN,
-NR^{a}₂, -C₁₋₄-alkyl-NR^{a}₂,
-OR^{a}, -C₁₋₄-alkyl-OR^{a}
-SR^{a}, -C₁₋₄-alkyl-SR^{a},
with each R^{a} being selected independently from each other from H, a C₁₋₄ alkyl, a C₂₋₄ alkenyl, or a C₂₋₄ alkynyl,
wherein the -C₁₋₄-alkyl, -C₂₋₄-alkenyl or -C₂₋₄-alkynyl is unsubstituted or substituted with -F,

7. The compound for use in the treatment of cancer according to any one of claims 5 or 6, wherein
R^{3a} is selected from -C₁₋₂-alkyl, -O-C₁₋₂-alkyl, -F, -Cl, -Br, -I, -NH₂, particularly -O-C₁₋₂-alkyl, -Cl, and -NH₂, and/or
R^{3b} and R^{3c} are independently selected from -C₁₋₂-alkyl, -F, -Cl, -Br, -I, particularly methyl and -Cl, more particularly -Cl.

8. The compound for use in the treatment of cancer according to any one of the preceding claims, wherein the linker L has a length that corresponds to the length of a linear hydrocarbon chain comprising 12, particularly 8, more particularly 6 carbon atoms.

9. The compound for use in the treatment of cancer according to any one of the preceding claims, wherein L is a linker comprising one or more moieties, particularly 1 to 4 moieties, independently selected from -C₁₋₆-alkyl, -O-, -NR⁴- and -C(=O)-, wherein
R⁴ is H or -C₁₋₄-alkyl.

10. The compound for use in the treatment of cancer according to any one of the previous claims, wherein L is selected from -C₁₋₆-alkyl-, -NR⁴-C₁₋₆-alkyl-, -C(=O)-C₁₋₆-alkyl-, -C(=O)-NR⁴-C₁₋₆-alkyl-, -NR⁴-C(=O)-C₁₋₆-alkyl-, -[O-C₁₋₆-alkyl]ₙ-, -C₁₋₆-alkyl-[O-C₁₋₆-alkyl]ₙ, wherein
R⁴ is H or -C₁₋₄-alkyl, particularly H, methyl or ethyl, and
n is 1, 2 or 3, particularly 1 or 2.

11. The compound for use in the treatment of cancer according to any one of the previous claims, wherein L is selected from -C₁₋₆-alkyl-, -C(=O)-NR⁴-C₁₋₆-alkyl-, -NR⁴-C(=O)-C₁₋₆-alkyl-, particularly from -C₁₋₆-alkyl-, -C(=O)-NR⁴-C₁₋₆-alkyl-, wherein
R⁴ is H, methyl or ethyl, particularly H.

12. The compound for use in the treatment of cancer according to any one of the previous claims, wherein the cancer is selected from leukemia, lymphoma and myeloma, particularly leukemia and myeloma.

13. The compound for use in the treatment of cancer according to any one of the previous claims, wherein the cancer is selected from acute lymphoblastic leukemia (ALL), acute myelogenous leukemia (AML), chronic lymphocytic leukemia (CLL), chronic myelogenous leukemia (CML), acute monocytic leukemia (AMoL), hairy cell leukemia, prolymphocytic leukemia (PLL), multiple myeloma (MM).

14. The compound for use in the treatment of cancer according to any one of the previous claims, wherein the cancer is selected from acute myelogenous leukemia (AML), chronic myelogenous leukemia (CML) and multiple myeloma (MM).

15. The compound of formula (1) for use as a CD93 inhibitor, particularly as an inhibitor of the CD93 intracellular domain.
